# EUROPEAN PATENT SPECIFICATION

(11) **EP 3 304 606 B1**
(45) Date of publication and mention of the grant of the patent: **09.01.2019**
(21) Application number: 16729208.5
(22) Date of filing: 27.05.2016
(51) Int. Cl.: H01L 41/04, A61M 25/00

(54) **CONTROL OF ACTUATOR DEVICE BASED ON AN ELECTROACTIVE POLYMER**
STEUERUNG EINER AKTUATORVORRICHTUNG AUF BASIS EINES ELEKTROAKTIVEN POLYMERS
CONTRÔLE D'UN DISPOSITIF D'ACTIONNEUR À BASE DE POLYMÈRE ÉLECTROACTIF

(30) Priority: 03.06.2015 EP 15170384
(43) Date of publication of application: 11.04.2018
(73) Proprietor: Koninklijke Philips N.V., 5656 AE Eindhoven (NL)
(72) Inventor: HENDRIKS, Cornelis Petrus, 5656 AE Eindhoven (NL); JOHNSON, Mark Thomas, 5656 AE Eindhoven (NL); FRANKLIN, Steven Ernest, 5656 AE Eindhoven (NL); VAN DEN ENDE, Daan Anton, 5656 AE Eindhoven (NL); VAN DE MOLENGRAAF, Roland Alexander, 5656 AE Eindhoven (NL)
(74) Representative: Marsman, Albert Willem
(86) International application number: PCT/EP2016/061960
(87) International publication number: WO 2016/193131

(56) References cited:
- WO-A1-00/74153
- WO-A1-2009/015151
- WO-A2-2007/090621
- JP-A- 2003 052 184
- US-A1- 2009 047 197

## Description

### FIELD OF THE INVENTION

This invention relates to the use of and to methods of controlling devices comprising electroactive materials for friction control. The invention also relates to devices comprising electroactive materials that are capable of being used or controlled for friction control. The invention further relates to computer program products related to the use and methods.

### BACKGROUND OF THE INVENTION

Electroactive materials (EAM) are materials that show mechanical deformation when electrically driven. Depending on the material in question, such driving can be in the form of subjecting the EAM to an electric field or to an electrically generated force by means of a suitable control signal. Certain classes of these EAMs also exhibit the converse effect, i.e. they can provide an electrical signal when subjected to mechanical deformation. The exact mechanism by which an EAM provides the above effects is dependent on the material of choice. Because of the above effects, the most common applications of such EAMs are in actuators and/or sensors.

Electroactive polymers (EAP) are an emerging class of materials within the field of EAMs. EAPs combine their favourable actuation-response properties with a number of advantageous engineering properties, therewith allowing use in new application areas. Thus, an EAP generally exhibits a relatively large deformation and force in a small volume or thin form factor, compared to common other mechanical actuators or actuators based on inorganic EAMs. EAPs also give noiseless operation, accurate electronic control, fast response, and the possibility of high resolution and cyclic actuation with a large range of possible actuation frequencies, such as 0 - 20 kHz. And all of these properties come with easy manufacturing into various shapes using well established methods allowing easy integration into a large variety of systems.

The performance and particular advantages of EAPs, which significantly improved over the last ten years, give rise to use in new applications. An EAP device can be particularly advantageously used in any application in which a small amount of movement of a component or feature is desired. Similarly, the technology can be used for sensing small movements.

### SUMMARY OF THE INVENTION

When a deforming EAM actuator is in contact with another surface, its movement can be restricted by static friction (stiction) or dynamic friction. Stiction can occur due to local surface roughness, dirt or wear particles, or other adhesion phenomena. Stiction is a risk especially in layered EAM systems and actuators and/or those which move around corners.

A known solution to reduce static friction (stiction) or dynamic friction in general is to apply normal or lateral vibrations to one of a set of contacting surfaces using an external actuator. However, this adds complexity to an actuator device. Alternatively, the friction surface profile itself can be changed by means of EAM actuators as taught by WO 2007/90621 A2 or US 2009/047197 A1, for example.

WO 00/74153 A1 discloses driving an EAM actuator (piezoelectric micromotor) by application of orthogonal actuation and friction control vibration signals, the frequency of the friction control signal being equal to or half of the actuation signal.

There is therefore a need for methods and devices with which a reduction in friction or else a controllable amount of friction can be realized and which can be implemented in a simple manner and with low cost.

It is an object of the invention to fulfill the aforementioned need. This object is achieved with the invention as defined by the independent claims according to which there is provided a method of actuation and an actuator device, as well as a computer program product for implementing the steps of the method. The dependent claims provide advantageous embodiments. Features, their advantages or problems they solve described for the method can be used to define corresponding features for the device and vice versa unless technically impossible.

In the invention the control signal is a signal to drive the EAM to deform. The control signal includes, at least within part of a drive period, the actuation signal and the vibration signal. The actuation signal is used to control the overall level of deformation of the EAM and therefore the actuation member comprising that EAM. This provides an actual desired actuation (output) of the device. The vibration signal is used to introduce a vibration of that same actuation member caused by vibrational deformation of the EAM for the purposes of reducing friction before or even during actuation. In this way, friction of an actuator device may be reduced without using an external actuator, saving space and reducing complexity as well as enabling improved use functions. The vibration can be used to help the actual actuation or to help achieving the desired actuation. The friction control also may be used to enable the actuator to be retained in set positions by removing the vibrational signal (and thereby increasing friction).

The actuation of the actuation member has a maximum first actuation frequency. This may for example be of the order of Hz or tens of Hz or hundreds of Hz. The actuation may be essentially static, i.e. a change from one actuation position to another at any arbitrary time. The vibration signal has a frequency greater than the first actuation frequency. The vibrations may be at hundreds or thousands of Hz or higher frequencies. They are used to assist the deformation at the lower frequency. The vibration frequency is for example at least ten times the maximum actuation freqeuncy, or even at least 100 times the maximum actuation frequency.

Any signal in the invention preferably is an electrical signal such as a voltage signal or a current signal. This may depend on the actual electroactive material used in the device. Field driven or capacitance driven EAMs may require voltage signals while ion diffusion driven EAMs may require current driving.

The control signal is generated such that in a driving period there is an actuation signal and a vibration signal. The actuation signal and the vibration signal can be provided to the electroactive material in a time sequential way not overlapping in a time. The actuation signal and the vibration signal may also partly or completely overlap in time. In that way friction control during actual actuation of the device is enabled.

The actuation signal and vibration signal can be separate signals or superimposed signals that can be both provided to the same part of the electroactive material. This could be done with one and the same electrode arrangement. This configurations reduces complexity and saves space.

The actuation signal and vibration signal can also be separate signals provided to different parts of the electroactive material. This may be in time overlapping way or not. There may be one part specifically defined for the friction control and another part for providing the main part or all of the desired actuation. The actuation member can be tailored towards such configuration to provide optimum response for both functions. The different signals can in this case be provided using different electrode arrangements. One of these can be optimized for receiving and treating the higher frequency vibration signal with low loss. Hence friction can be modified or controlled during actuation.

The actuation signal can comprise a non-oscillating signal. This can be a DC signal. The actuation signal can be a DC signal. With DC is meant a signal that varies more slowly than the vibrational signal or that varies not at all. It may thus have a lower frequency than the vibrational signal. The DC signal level may be linearly or otherwise increasing or decreasing. Note however that the actuation signal can also be varying signal or even oscillating signal in case an oscillating actuation is needed.

A vibration signal is a part of the control signal and causes the actuation member to vibrate, but is not meant to provide an actual actuation output of the device. Hence its signal amplitude or level can be chosen to be smaller than that of the actuation signal preferably the amplitude or level is smaller than 20%, smaller than 10% or smaller than 5% or smaller than 1% of that of the actuation signal.

The actuation signal can comprise or consist of a pulse signal where the pulse signal is a single pulse, a multiple pulse sequence, or repetitive pulse sequence. In case of the multiple pulse or repetitive actuation pulse sequence, the frequency of such sequence is lower than the frequency of the vibration signal. The controller can be configured to be capable of providing a pulse wherein part of or the entire pulse duration the signal level decreases or increases with time, or even oscillates in time with a frequency lower than the AC signal frequency. Preferably this signal level is only decreasing or increasing (e.g. linearly) or substantially constant (pulse serves as DC signal for a fixed period of time) such that the actuation signal during the pulse duration serves as a general actuation signal. In the aforementioned cases the actuation signal can be at least partly superimposed with the AC signal. Thus, during at least part of the actuation signal pulse duration there is then simultaneously provided at least one period of the AC signal.

The vibration signal is a deliberately applied signal, and is not merely a noise or other spurious signal. The vibration signal can comprise a vibration signal frequency which is chosen to be: < 1 MHz, <100kHz, <10kHz, or <1 kHz. The vibration signal may for example have a frequency below 1 kHz.

Below 1 kHz can for example be suitable for generating 1-10 µm out of plane vibrations of an actuator member. Higher frequencies may be used typically for smaller out of plane vibrations. Between 1 kHz and 1 MHz vibrations of a small number of micrometers may be obtained, and above 1 MHz (ultrasound) sub-micrometer out of plane vibrations may be obtained.

The vibration signal can be higher than 250 or higher than 500 Hz to better separate the actuation signal from the vibration signal of the invention.

The actuation signal can be an oscillating signal comprising an actuation signal frequency and the vibration signal comprises a vibration signal frequency that is higher than the actuation signal frequency.

The actuation signal frequency can be a highest actuation signal frequency. The actuation signal frequency may be lower than the vibration signal frequency by a factor of 2, 5, 10, 20, 50, 100, 200, 500, 1000 or even more. Preferably, the actuation signal frequency is now below 500 Hz, below 200 Hz, below 100 Hz or even below 50 Hz.

The vibration signal can comprise at least one vibration signal frequency that is equal to a resonance frequency or eigenfrequency of the actuation member. This provides a low power driving while having good frictional control properties.

The invention can comprise that:
in a first operating mode, supplying the actuation signal and the vibration signal to the electroactive material; and
in a second operating mode, supplying only an actuation signal and no vibration signal to the electroactive material.

These two modes define a low friction mode and a high friction mode.

The amplitude of the actuation signal may be selected to provide a desired general level of deformation of the electroactive polymer. The amplitude of the vibration signal may be selected to provide a desired general level of vibration to the deformation of the electroactive polymer. The frequency of the AC component may be selected to induce a resonant vibration or not.

The method can be implemented in a computer program product comprising computer code stored on a computer readable medium or downloadable from a communications network, the computer code, when executed on a computer, implementing the method of the invention. This can comprise controlling a controller to perform the steps of any of the methods. The computer readable medium can be a data storage medium as known in the art such as computer memory or storage disk of any kind. The communications network can be a wired or wireless network of any kind such as WAN, LAN etc.

The invention can be embodied in a device comprising:
an actuation member comprising an electroactive material capable of deforming upon driving with a control signal;
a controller configured to implement the steps of the method of the invention.

Thus, e.g. the controller may be adapted to provide the actuation signal with selectable amplitude and/or pulse duration. This selectable amplitude may be used to control the general level of deformation and the time of the deformation, and thereby provide an analogue general actuation. This may be the actuation signal.

The controller may be adapted to provide the vibration signal with selectable amplitude and or duration. This selectable amplitude may be used to control the amplitude of the vibrations, and therefore the friction level, and thereby provide an analogue friction control. The duration is important to control the duration of friction.

The controller may be adapted to provide an vibration signal with selectable frequency. This selectable frequency may be used to induce resonance in the vibrations based on the mechanical characteristics of the EAM.

Also the controller can be adapted to be operable in a first mode in which the vibration signal and the actuation signal are provide to the electroactive material. This may be superposed on the actuation signal (e.g. DC signal) and a second mode in which no vibration signal is superposed. In this way, the friction can be controlled, for example switching the device between a high friction state and a low friction state. A high friction state may correspond to a static position of the actuator device or catheter device, and the low friction state may correspond to adjustment of the device shape or catheter position.

The device can further comprise an electrode arrangement configured to receive the actuation signal and the vibration signal from the controller and therewith to supply it to at least part of the electroactive material. This provides a simple construction with only one electrode arrangement for both signals. Both signals can now also be applied to the same parts of the electroactive material.

The device or even the actuation member can further comprise:
a first electrode arrangement configured to receive the actuation signal and therewith to supply it to a first part of the electroactive material; and
a second electrode arrangement configured to receive the vibration signal and therewith to supply it to a further part of the electroactive material that is different from the first part of the electroactive material. This configuration allows provision of the different signals to different (possibly optimized parts) of the electroactive material.

The device can have the actuation member comprising a first surface which is exposed such that it is capable of being in frictional contact with a second surface of a substrate. Herein the vibration signal is for modification or reduction of friction between the first surface and the second surface. This is useful to control friction in case the device is to be moved along an external surface in use.

In one set of examples of the device comprises the substrate against which the actuation member is positioned, wherein the vibration to the deformation of the electroactive material is to reduce friction between the substrate and the actuation member. This friction control may then be used to control the way the device itself deforms.

The device can comprise the substrate and the substrate is then arranged such that with or without actuation of the actuation member, friction between the first surface and the second surface can be modified with the vibration signal.

The device can have a substrate which comprises a further actuation member as defined in any one of the previous claims which can be driven by the control signal or another control signal. In this way stacked actuation members can benefit from reduced friction when both are activated.

The device can comprise:
a body for guided movement along an internal guide or an external conduit, the body comprising the actuation member and optionally the internal guide or external conduit comprising the substrate; or
an internal guide or external conduit for guided movement of a body, the internal guide or external conduit comprising the actuation member and optionally the body comprising the substrate.

The body can be catheter or part of a catheter. It may also be another device for entering a human or animal body such as an endoscope etc. In this case the friction can be controlled during the movement along the internal guide (which may be a guide wire) or the external conduit. The external conduit can be a tube like member.

In another set of examples, including the catheter example defined above, the device is to be moved along an external surface in use, wherein the vibration to the deformation of the electroactive polymer is to reduce friction between the electroactive polymer and the external surface. The friction control is then used to assist movement of the device along another surface or structure.

The method may be applied to a catheter, and the actuator is then for providing electrically controllable friction. It may however be applied to a large number of other possible actuator devices, some of which are discussed further below.

As mentioned above, the substrate may itself be part of the device or it may be external to the actuator device.

The device can comprise or consist of a catheter, and the actuator is for providing electrically controllable friction. Alternatively or additionally, the device may comprises a variety of other possible actuator devices, some of which are discussed further below.

The catheter has a layer to which a vibration signal may be applied to provide a reduction in friction, which e.g. can be used to assist the movement of the catheter along a guide wire and/or the movement of the catheter along a conduit such as a blood vessel or artery.

The central space within a tube-shaped body part or organ, such as a blood vessel or the intestine.

Examples of field-driven EAPs are dielectric elastomers, electrostrictive polymers (such as PVDF based relaxor polymers or polyurethanes) and liquid crystal elastomers (LCE).

Examples of ionic-driven EAPs are conjugated polymers, carbon nanotube (CNT) polymer composites and Ionic Polymer Metal Composites (IPMC).

Field-driven EAP's are actuated by an electric field through direct electromechanical coupling, while the actuation mechanism for ionic EAP's involves the diffusion of ions. Both classes have multiple family members, each having their own advantages and disadvantages.

This invention is of primary interest for field-driven EAPs as they have faster response time. However, the concepts may be applied to ionic-drive EAPs as well.

### BRIEF DESCRIPTION OF THE DRAWINGS

Examples of the invention will now be described in detail with reference to the accompanying drawings, in which:
Figure 1 shows a known electroactive polymer which is not clamped;
Figure 2 shows a known electroactive polymer which is constrained by a backing layer;
Figure 3 shows a first example of control approach for an electroactive polymer;
Figure 4 shows a second example of control approach for an electroactive polymer;
Figure 5 shows a third example of control approach for an electroactive polymer;
Figure 6 shows a fourth example of control approach for an electroactive polymer; and
Figure 7 shows another possible control voltage waveform.
Figure 8 shows a first example of friction reducing layer applied to the outside of a catheter;
Figure 9 shows a second example of friction reducing layer applied to the outside of a catheter;
Figure 10 shows a catheter having a friction reducing outer layer with a first electrode design;
Figure 11 shows a catheter having a friction reducing outer layer with a second electrode design;
Figure 12 shows a catheter having a friction reducing outer layer with a third electrode design; and
Figure 13 shows how different layer designs give rise to different displacement versus drive voltage functions.

### DETAILED DESCRIPTION OF THE EMBODIMENTS

The invention pertains to friction control between surfaces of an actuator device using specific actuation of electroactive materials for causing at least one of the surfaces to vibrate. The invention allows such vibration to occur even during actual actuation of the electroactive material.

In the invention a number of terms/features have the meaning as defined below.

An actuation member is a part of the device which comprises an EAM. The actuation member can consist of the EAM. There may be more than one EAM in the member. By driving the EAM, the actuation member can provide an actuation output.

Electroactive material is a material that is capable of mechanical deformation when subjected to an electric field or an electrically generated force. Mechanical deformation can include a change of shape and/or location. Specific examples and classes of materials are given herein below.

An electrode arrangement can be an ensemble of one or more (preferably two or more) electrodes arranged such that they enable supply of an electrical control signal to at least part of an EAM in the actuation member of the device. The electrodes of an electrode arrangement can be attached to the EAM, but that is not always needed. In case of dielectric elastomer EAMs, such attachment is preferred.

The possible implementation of the invention will be described with respect to a number of examples. However other examples falling within the scope of the claims can be thought of.

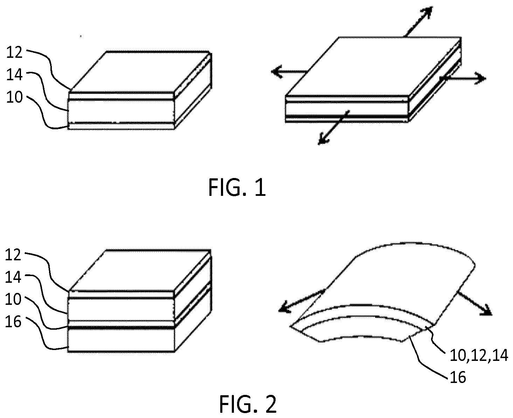

Figures 1 and 2 show two possible operating modes for a known EAP device.

The device comprises an electroactive polymer layer 14 sandwiched between electrodes 10, 12 on opposite sides of the electroactive polymer layer 14.

Figure 1 shows a device which is not clamped. A voltage is used to cause the electroactive polymer layer to expand in all directions as shown.

Figure 2 shows a device which is designed so that the expansion arises only in one direction. The device is supported by a carrier layer 16. A voltage is used to cause the electroactive polymer layer to curve or bow.

The nature of this movement for example arises from the interaction between the active layer which expands when actuated, and the passive carrier layer. To obtain the asymmetric curving around an axis as shown, molecular orientation (film stretching) may for example be applied, forcing the movement in one direction.

The expansion in one direction may result from the asymmetry in the EAP polymer, or it may result from asymmetry in the properties of the carrier layer, or a combination of both.

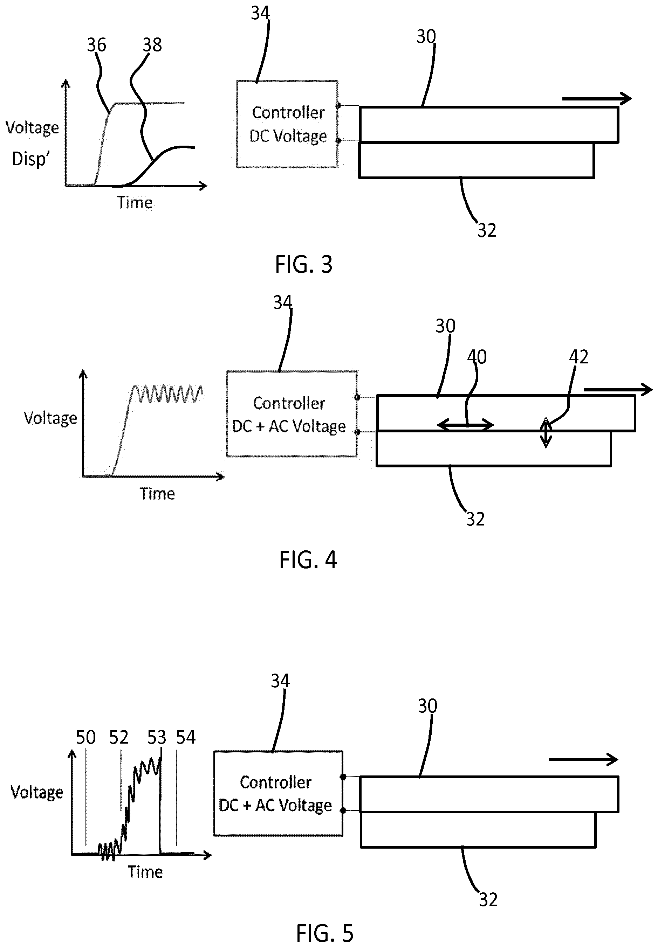

Figure 3 shows a design of an actuator device in which an electroactive polymer (EAP) layer 30 is mounted to a substrate 32, and in which the movement of the layer 30 is in-plane, i.e. a plane defined by a contact surface between the members 30 and 32. This may be achieved by mounting the layer 30 to the substrate at one side (e.g. the left in Figure 3), and otherwise the EAP layer 30 is freestanding as in Figure 1. When the layer 30 deforms (as shown by the arrow in Figure 3) there is friction between the layer 30 and the static substrate 32.

A controller 34 is used to apply an actuation signal in the form of a direct current (DC) voltage across the EAP layer 30, and the voltage-vs-time profile shown as 36 corresponds to a step change in applied voltage at a certain time. The control signal is provided to the EAP layer by means of an electrode arrangement that includes two electrodes one on either side of the EAP layer and the Controller is connected to supply its control signal to one or both electrodes. The electrodes are in the plane of the movement indicated and are not shown for reasons of clarity.

The friction (frictional resistance) between the layers resists the relative sliding movement until the frictional force is overcome. Thus the system may have an intrinsic delay of mechanical output upon actuation. This delay can be permanent (indefinite delay time) if the actuation is insufficient to cause an actuation force suitable to overcome the friction force, but can also be non-permanent (definite delay time) if the actuation force needs time to build up after application of the actuation signal. The latter situation is shown in Figure 3. Thus, if the voltage-vs-time profile shown is applied, the displacement (Disp') is delayed and this is shown in the displacement ("Disp' ") plot 38 which has a delay before the movement starts as well as a relatively slow rise because the friction has to be overcome. In this way, the friction (in particular stiction) functions as a delay mechanism of the device. Stiction can occur due to local surface roughness, dirt or wear particles, or other adhesion phenomena. Stiction is a risk especially in layered systems and actuators which slide against other surfaces, for example moving around corners.

A known solution to reduce static friction (stiction) or dynamic friction in general is to apply normal or lateral vibrations to one of the contacting surfaces using an external actuator. This is for example disclosed in Teidelt, E. et al., 2012, "Influence of Ultrasonic Oscillation on Static and Sliding Friction", Trib. Lett., Vol.48, 51-62. However, this requires additional components, such as an external actuator, to reduce friction and this limits applications of the EAP based device with respect to the desire of using it in confined spaces for which they typically are useful because of the their small form factor achievable.

The invention is based on the application of a relatively high frequency vibration signal to the EAP causing a vibration of the EAP containing member or surface.

Thus, as a modification of the example of Figure 3, the controller 34 is now configured to also generate and supply an alternating current (AC) signal (vibrational signal) to the EAP during some vibration period. The vibration period is in this case at least a part of the actuation period and in this case the vibration signal can be seen to be a small amplitude sine wave signal provided within a vibration period that is the part of the actuation period in which the actuation signal is at constant level. In this case this vibration signal is provided to the electrodes that are also used for the actuation signal and hence both signals are provided to the EAP as the result of a superposition and also at the same location of the EAP as shown in Figure 4. Note that for clarity reasons the actual electrodes have not been drawn.

In the example, the AC signal part results in surface vibrations which are superimposed on the nominal actuator displacement. The surface vibrations reduce the contact area ("floating contact") and loosen trapped particles or mechanically interlocked surface defects (e.g. local surface roughness peaks or scratches). The vibrations may be manifested as in-plane (arrow 40) and/or normal (arrow 42).

The control signal (drive waveform) shown in Figure 4 comprises the superposition of a DC component (actuation signal) for controlling the general level of deformation (i.e. a so called desired useful actuation of the device) of the electroactive polymer and an AC component (vibrational signal) for introducing a vibrational deformation (i.e. an additional actuation with the effect to influence the delay time for reaching the useful actuation) that adds to the general level of deformation of the electroactive polymer. The AC component is shown as applied once the DC level has reached its new level, but it may also be applied during the ramp part of the DC signal or to both parts and even after removal of the actuation DC signal thus facilitating return of the EAP to its non-actuation state.

In the example of Figure 4, the controller 34 is able to deliver an AC and a DC voltage. This approach does not need additional structural modifications to the device.

The vibrational signal and the actuation signal do not need to be supplied to the same part of the EAP per se. Thus separation of locations on the EAP for providing the actuation signal and the vibration signal to, would be allowed as long as the vibration signal provided to the EAP causes sufficient vibration of the EAP containing member such that the delay time upon actuation is reduced in comparison to the situation of similar actuation without the vibration signal. This separation of location can include that a further electrode arrangement is needed in the example of Figures 3, 4 ad 5. Such separation can be advantageous in situations where design of a device results in an optimum actuation of the device occurring using a first location of signal provision to the EAP and optimum vibration requires a second location that differs from the first location for provision of the vibration signal to the EAP.

The EAP actuator may be a single layer device or a multilayer device (not shown). The EAP actuator in this case is shown as a sliding actuator, but can also be a bending actuator, which may upon bending also experience sliding induced friction.

For the example of Figure 4, it is assumed that the EAP layer deforms relatively slowly compared to the frequency of the AC signal (vibrational signal). Hence in Figure 4, once the actuation signal driving voltage increases, the EAP is deforming during the entire time period depicted in the graph (as can be seen in Figure 3), and there are active vibrations during the deformation induced by the AC component. In general, the higher the amplitude of the AC signal, the lower will be the friction as the vibrations are larger. In this way, the displacement curve can be made steeper and the actuator can reach its final configuration more quickly. In turn, this enables higher frequency actuation operation of the device.

Figure 5 shows an actuator with a high frequency AC signal added to a DC actuation signal to enable slip when the actuator moves from one position to a next position. The next position is held by removal of the applied voltage due to return of the friction and therefore sticking function. This gives the actuator a bistable functionality, in that two different positions may be adopted with no drive signal applied.

In this example, it is assumed that the EAP deforms somewhat faster (relative to the frequency of the AC signal) than in the example above.

As shown in the voltage vs. time profile in Figure 5, the driving of the device starts with an AC voltage with only a small DC offset, between time points 50 and 52 (i.e. the signal oscillates effectively between zero voltage and amplitude voltage), so that there will result a vibration around an almost non-actuated state, i.e. the device will vibrate between the non-actuated state and a small amplitude actuated state. This will result in a reduction of friction and prepare the electroactive polymer layer for a smooth actuation movement, which occurs as soon as the driving voltage increases. Hence, delay can be reduced to a minimum.

The EAP layer then continues to deform during the next time period depicted in the graph, between time points 52 and 53 where there are active vibrations during the deformation induced by the AC component superposed on the rising DC voltage level.

Finally, following a short period where the AC signal is superposed upon an essentially constant DC level, to allow for any delay in the movement of the electroactive polymer layer in reaching its final state (There will be an intrinsic delay between the mechanical deformation and time of providing the control signals due to the intrinsic material EAP properties as well as a specific design of a device), the voltage is removed at time 53 which, if the residual friction is sufficient, will result in a second stationary state being retained at time 54. Subsequently the device can be reset by applying only the small AC signal of time period 50 to 52, or another small signal AC signal, to overcome the friction and bring the device back to its original state.

Hence the invention allows construction and operation of a device that has two (or more) stable states with a reset possibility. In such cases, like the example of Figure 5, it may be advantageous to reduce the AC signal amplitude during its supply to the EAP (decaying AC signal) slowly to allow the device to settle into its most stable (highest friction) state.

In general, it is again the case that the higher the amplitude of the AC signal, the lower will be the friction as the vibrations are larger.

This bistable functionality may for example be used to save power in that a drive signal can be removed once the device is held in a stable state, and positive driving is only needed during movement of the actuator.

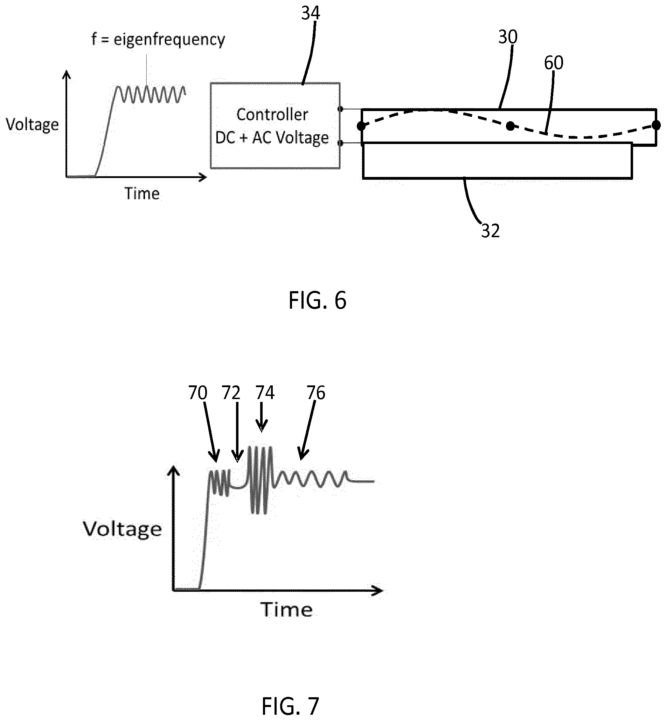

Figure 6 shows another variation, which may be considered to be a low power implementation of the invention.

Again, a high frequency AC signal as the vibrational signal is added to a DC driving signal as the actuation signal, as in Figure 4. However, in this example, the frequency of the AC signal is selected to be one of the eigenfrequencies of the EAP actuator or the member comprising the EAP. This leads to standing wave resonance vibration as e.g represented by the standing wave 60. The advantage is that the actuator, once it is driven in resonance, can achieve higher strains at the same applied AC voltages, so it is more capable of overcoming friction with lower amplitudes of a vibration signal.

The disadvantage is that the eigenfrequency is specific for each actuator design and the value of the eigenfrequency is dependent on boundary conditions acting on the EAP. Also, the actuator must be brought into resonance while being clamped by friction, which needs a high initial input energy. Eigenfrequencies can be determined for a device using appropriate calibration procedures (the person skilled in the art will know how to measure such frequencies) and stored in lookup tables for use by the controller if needed.

For the actuator in Figure 6, some degree of tuning of the friction control can be achieved by adapting the frequency to different resonance states (i.e. longitudinal, lateral and thickness fundamental resonance frequencies and higher harmonics).

The actuator may be initially driven using a large AC amplitude to reduce friction, and once in resonance a lower voltage amplitude can be applied, to lower the power consumption and increase the lifetime of the actuator. The impedance value of the actuator may be used to tune the AC amplitude, in a feedback loop. Those skilled in the art will know how to implement such feedback loops according to electronics principles.

This implementation of the invention provides electronically controlled surface friction.

It will be seen from the various examples above that the controller may be able to selectively apply the AC component and it may be able to adjust the amplitude and/or frequency of the AC component over time continuously or stepwise, as well as being able to adjust the DC component to different levels.

Figure 7 shows an exemplary drive scheme which combines these abilities. A control signal with AC ripples of different frequencies and amplitudes is used to electronically control the friction coefficient of the EAP actuator surface. Time period 70 provides a low friction state with relatively low amplitude high frequency AC component. Time period 72 provides a sticking state with no AC component. Time period 74 provides a near-zero friction state with relatively high amplitude high frequency AC component. Time period 74 provides a medium friction state with relatively low amplitude and also low frequency AC component.

In the examples above, the device has a substrate 32 against which the EAP 30 is positioned, so that it is an internal friction within the device that is controlled. This is of particular interest for an actuator which deforms in-plane as shown or for example for a multilayer bending actuator where there may be friction between different layers during deformation.

However, it is also possible that the device is to be moved along an external surface in use, wherein the vibration to the deformation of the electroactive polymer is to reduce friction between the electroactive polymer (member) and the external surface (which is not part of the device itself). The actuator may be a bending, twisting or in-plane deforming actuator.

There are various examples of device which is moved in use and may experience friction against an external component. One example which will now be discussed is a catheter or any other guide wire type of device. The friction between a catheter and the wall of the vessel through which it passes can lead to stresses and strains that can cause damage to the vessel. In certain vascular catheters, sliding friction is also present between the guide wire or manipulation wire and the wall of the inner lumen of the catheter, which pulls on the guide wire. A high and/or unstable frictional resistance between the guide wire and the catheter lumen causes reduced tactile feedback, lag and high insertion forces at the entry point, which may cause the catheter to buckle or cause problems with retraction of the catheter.

It is well known that surface patterning can in some circumstances lead to friction reduction in lubricated contacts. It is also known that out-of-plane oscillation can be used to reduce friction (as mentioned above). Dynamic surface deformation is known to reduce the friction by reducing the "stick" part of stick-slip sliding, for example as shown in the article "Influence of Ultrasonic Oscillation on Static and Sliding Friction" of E. Teidelt et al., referenced above. The coefficient of friction can be made to reduce by orders of magnitude when oscillations of the surface are applied. This is especially valid at low sliding speed (0 - 10 mm/s) which is applicable to the movement of invasive devices such as catheters.

A more conventional approach to friction reduction between a catheter outer surface and vessel wall is to coat the catheter with a low friction coating, i.e. a slippery hydrophilic surface which reduces friction or a microstructured surface aimed at reducing friction. However, once the catheter is at the desired position it should remain there in order to carry out the required treatment precisely; a very low friction resistance causes difficulty in maintaining the correct position in the vessel.

Also important is that physicians desire a certain amount of tactile feedback when inserting the catheter. This amount may differ per physician and also vary for different parts of the procedure. If physicians could control the friction properties of the catheter they could control the amount of tactile feedback to make it suitable for their needs.

The vibration control as explained above (which does not itself require a DC component) may be applied to the inner surface (contacting a guide wire) or outer surface (contacting a vessel wall) of a catheter so that the shape can be deformed out of plane to generate small oscillations along the surface. The friction can then be reduced, for example relative to the vessel in which the catheter is inserted, when needed and it can be restored to a higher friction when necessary, for example when the device is at its desired location and must be held in place.

To implement this approach for controlling the friction between the catheter and an external vessel in which the catheter is inserted, an EAP is used to form a material film to the outer surface of the catheter which film can be made to vibrate using the EAP. The film can be the EAP surface, but need not be that surface per se, as long as an EAP can be addressed to make that surface vibrate.

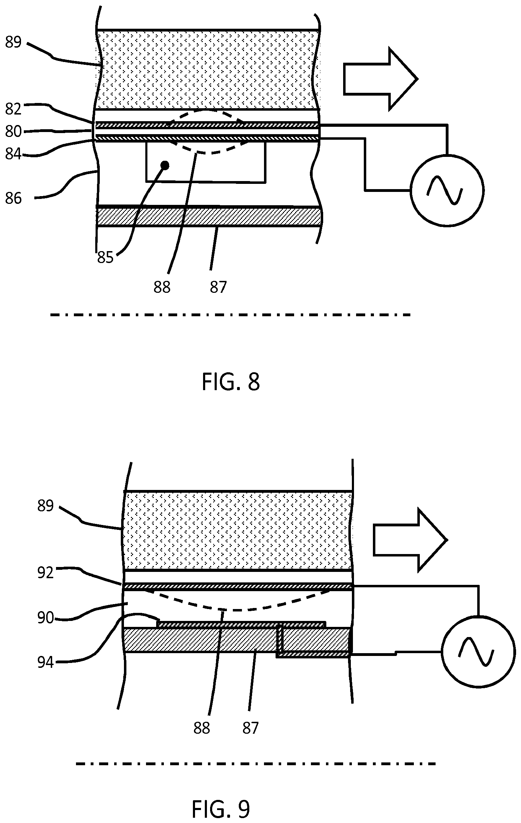

Figures 8 and 9 show a small portion of the length of a catheter and at one side only of the centerline of the catheter.

In one arrangement shown in Figure 8, the EAP layer 80 (and its electrode layers 82,84) is only partly adhered to the outer surface of a catheter because recesses 85 are formed in an outer layer 86 over the outside of the catheter lumen 87. In this way, part of the EAP layer is free to move and generate out of plane surface oscillations 88, providing dynamic surface texturing. This makes it possible to control and/or reduce friction along the whole length of the catheter shaft or a part of it where desired.

The shallow cavity 85 below the free part of the EAP layer means it is free to oscillate. The EAP layer will deform when a voltage is applied and cause an out of plane static deformation when a DC voltage is applied or an oscillatory motion when an AC voltage is applied.

Thus, DC or AC control is again possible, as well as AC superposed on DC as in the examples above. DC control will reduce friction to a certain extent by providing a micro-structured surface, and AC control will additionally induce vibrations which further reduce the friction.

The deformation may occur by bilayer bending or by a buckling type movement.

As in the examples above, the frequency and amplitude of the AC signal, as well as the level of a DC signal, and additionally the mechanical design, can give a high degree of tunability of the friction coefficient under many different circumstances.

The EAP layer may also be tuned to a desired Young's modulus (for example tunable towards the material properties of a catheter), minimizing the influence of the responsive material on the overall flexibility of the catheter. Thin EAP layers may be used.

To manufacture the example of Figure 8, a polymer coating 86 is applied which will act as a support structure, typically less than 100 µm thick. The cavities 85 are then machined or embossed and an adhesive is applied on the protruding parts of the surface (i.e. around the cavities). A thin layer of electroactive material 80 with applied thin film electrodes 82, 84 is attached to the support structure using the adhesive, for instance by rolling around the catheter and curing the adhesive. Alternatively, the electroactive material can be laminated onto a substrate of variable thickness to increase bending actuation (using bilayer bending).

The electrodes can be applied on either side of the film or alternating on the inside of the film in an interdigitated configuration. If necessary, a passive conformal coating may be applied on the surface to further adjust friction properties.

Electrode patterns may also be configured in specific patterns to create 2D arrays of dimples on the outer surface of the catheter.

The electrodes are connected and when a DC signal is applied, the EAP expands or contracts causing a deformation of the surface, thereby changing the real contact area between the catheter and vessel and influencing lubrication properties. When an AC voltage is applied the timescale of the actuation can be changed, causing a continuously changing surface topology. The frequency and voltage/amplitude can be adjusted to control the friction between the surfaces.

Figure 9 shows an alternative in which a thick, softer EAP layer 90 can be deformed out-of-plane by electrostriction to give a switchable surface topology. Such soft electrostrictive polymers for example include silicone and polyurethane elastomers and acrylates. The layer is again formed between electrodes 92, 94 and the three layers are provided over the catheter outer wall 87.

The use of a friction control actuator at the outer surface of the catheter has been described above. A similar approach may be taken for the inner surface of the catheter which is in contact with the guide wire. The guide wire surface (typically Nitinol and coated with a thin PTFE layer) is much harder, so the electroactive polymer surface may have to be coated with a harder material to increase the effect of friction reduction.

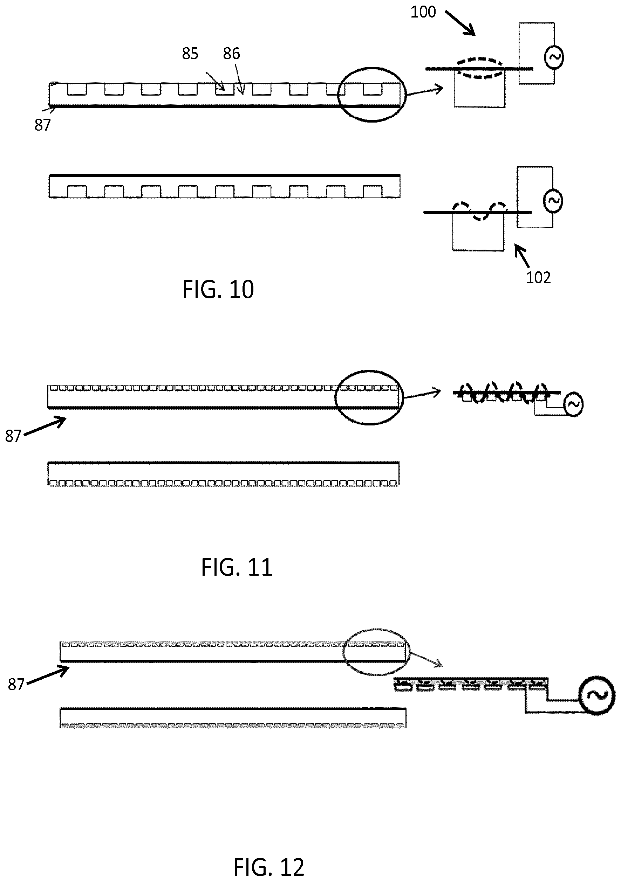

Figures 10 to 12 shows various different catheter designs.

Figure 10 shows a version based on Figure 8. The catheter has an outer lumen i.e. the catheter body 87, over which the outer polymer layer 86 is provided with its voids 85.

In this example, the EAP is controlled by planar electrodes on opposite sides of the EAP layer, as shown in Figure 8.

Figure 10 shows two possible drive schemes at different frequencies. The first resonant frequency gives rise to deformation shown by plot 100, with a half wavelength defined in each cavity. Plot 102 corresponds to higher resonance frequencies.

Figure 11 shows a design with interdigitated electrodes provided on a single side (the side facing the catheter wall) of the EAP layer. These comprise two comb electrodes with alternating comb fingers.

Figure 12 shows a design with one continuous electrode and one patterned electrode so that the deformation can be limited to defined zones of the overall EAP layer.

Each of these approaches may be used to introduce a wave like buckling shape, and it may be controlled to be static (with a DC voltage applied) or dynamic (with an AC voltage or an AC voltage superposed on a DC base level).

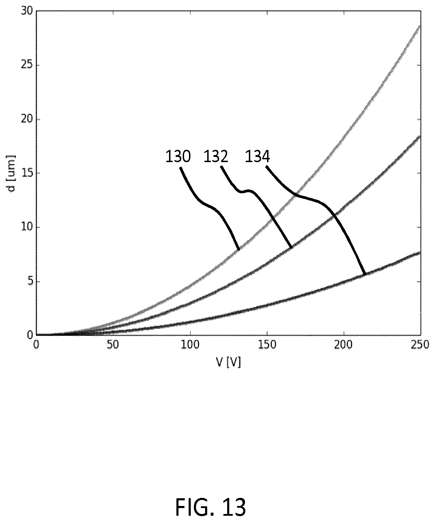

Typical static deformations which can be achieved using the design shown in Figure 10 are shown in Figure 13 for a 5 µm thick PVDF-TrFE-CFE bilayer on a plastic (i.e. Polyimide, PET, PC) substrate with three different thicknesses. The graph shows the displacement voltage function. Plot 130 is for a 2.5 µm substrate thickness, plot 132 is for a 5.0 µm substrate thickness, and plot 134 is for a 10.0 µm substrate thickness. The cavity width is 0.5 mm.

Apart from the voltage amplitude, the deflection will also depend on frequency of the applied voltage and on the proximity to the vessel wall. Deformations of 5 µm already give a huge effect on friction, depending on conditions such as stiffness, surface hydrophilicity, dry/lubricated, load and sliding velocity. Therefore, being able to control the surface topology can have huge influence on the friction properties of the catheter, allowing reduction of blood vessel damage, controllable haptic feedback and better positioning and holding.

This design (invention in general) may be applied to different types of catheter, such as vascular catheters or urinary catheters for use on human and/or enamel bodies.

In all examples above, the electrode arrangement may comprise electrodes on opposite faces of the electroactive polymer layer as shown above, for a field driven device. These provide a transverse electric field for controlling the thickness of the EAP layer. This in turn causes expansion or contraction of the EAP layer in the plane of the layer.

The electrode arrangement may instead comprise a pair of comb electrodes on one face of the electroactive polymer layer. This provides in-plane electric field, for directly controlling the dimensions of the layer in-plane.

For the examples above which make use of an AC component superposed on a DC drive level, a DC voltage of 200 Volts may give a 200 µm nominal displacement (e.g. a loaded actuator). For friction reduction, a 2 µm vibration may be desired in order to have an effective friction reduction, but not to disturb the application excessively. This would for example mean an AC ripple of 2 Volts (by linear interpolation), so the magnitude of the AC component is 0.01 times the DC component magnitude. The AC voltage magnitude may be taken to be the mean to peak amplitude (i.e. half of the peak to peak magnitude). The actual voltage levels needed in control signals depend on a number of parameters such as the EAP material in questions (organic or polymer EAM generally require higher amplitude signals than inorganic EAM); the thickness of EAM over which an electric field must be applied etc. Thoses skilled in the art will know how to adjust the levels to a device in question using theory of EAM materials and electric capacitor considerations as mot EAM are electrically isolating dielectric materials addressed with voltages applied to electrodes.

Noise levels in DC signals for EAPs are typically less than 1%.

Generally, the AC component magnitude is less than 10% of the DC component magnitude, and may be less than 5, or even less than 1% of the DC component. However, the AC component should have a magnitude greater than the noise level, for example greater than 10 times the noise level.

The AC ripple voltage may for example be between 1 and 5 Volts for a 150-250 Volt DC actuation. In general the AC actuation per unit of AC voltage driving amplitude increases with DC voltage so that a relatively larger AC component is needed for lower DC actuation.

For example, suitable combinations of signals may be:

| DC voltage | AC voltage amplitude |
|---|---|
| 50V | 20V |
| 100V | 10V |
| 150V | 5V |
| 200V | 2V |
| 250V | 2V |

In the invention, such as in the above examples, the actuation signal is a DC signal. Alternatively the actuation signal may be a varying level signal as long as the variation is slower than that of the vibration signal. The actuation signal can be a time increasing or not or a decaying signal linear or non-linear.

The invention will have its effect for a variety of electroactive materials. Thus although the examples have been described with reference to the EAPs, such materials can in fact be replaced with other electroactive materials. Hence, unless indicated otherwise, the EAP materials hereinabove can be replaced with other EAM materials. Such other EAM materials are known in the art and the person skilled in the art will know where to find them and how to apply them. A number of options will be described herein below.

Among the many EAM devices, a common sub-division is into those based on field-driven and ionic-driven EAMs. Field-driven EAMs are actuated by an electric field through direct electromechanical coupling, while the actuation mechanism for ionic EAPs involves the diffusion of ions. Both classes have multiple family members, each having their own advantages and disadvantages.

Many field driven EAMs, of organic or inorganic nature exist. For example, The EAM material can be a relaxor ferroelectric inorganic material. Such materials can have an electrostrictive constant that is high enough for practical use. The most commonly used examples are: lead magnesium niobate (PMN), lead magnesium niobate-lead titanate (PMN-PT) and lead lanthanum zirconate titanate (PLZT).

A special kind of EAM materials are organic electroactive materials OEAMs to which also Electroactive polymers (EAPs) belong. The organic materials and especially polymers are an emerging class of materials of growing interest as they combine the actuation properties with material properties such as light weight, cheap manufacture and easy processing. The actuation properties are often larger than those of their inorganic counterparts. Also, a number of the EAP materials are chemically tolerable for use with a human or animal body, something not always the case with the inorganic counterparts (e.g. Pb containing perovskites).

Examples of field-driven EAPs are dielectric elastomers, piezoelectric polymers, relaxor ferroelectric polymers, ferroelectric polymers, electrostrictive polymers (such as PVDF based relaxor polymers or polyurethanes) and liquid crystal elastomers (LCE). The dielectric elastomers strictly spoken are not field driven materials. Their response is based on force applied on them where the force is exerted by the electrodes carried by them. For the purpose of this invention they can however be included in the definition of EAP. Examples of ionic-driven EAPs are conjugated polymers, carbon nanotube (CNT) polymer composites and Ionic Polymer Metal Composites (IPMC).

Electro-active polymers include, but are not limited to, the sub-classes: piezoelectric polymers, electromechanical polymers, relaxor ferroelectric polymers, electrostrictive polymers, dielectric elastomers, liquid crystal elastomers, conjugated polymers, Ionic Polymer Metal Composites, ionic gels and polymer gels.

The sub-class electrostrictive polymers includes, but is not limited to:
Polyvinylidene fluoride (PVDF), Polyvinylidene fluoride - trifluoroethylene (PVDF-TrFE), Polyvinylidene fluoride - trifluoroethylene - chlorofluoroethylene (PVDF-TrFE-CFE), Polyvinylidene fluoride - trifluoroethylene - chlorotrifluoroethylene) (PVDF-TrFE-CTFE), Polyvinylidene fluoride- hexafluoropropylene (PVDF - HFP), polyurethanes or blends thereof.

The sub-class dielectric elastomers includes, but is not limited to:
acrylates, polyurethanes, silicones.

The sub-class conjugated polymers includes, but is not limited to:
polypyrrole, poly-3,4-ethylenedioxythiophene, poly(p-phenylene sulfide), polyanilines.

Additional passive layers may be provided for influencing the behavior of the EAP layer in response to an applied electric field.

Electrodes can be made of any electrically conducting material. Such materials include but are not limited to metals, electrically conducting organic materials such as organic polymers, composite materials comprising conducting particles in a matrix of e.g. polymeric material. Metals include Noble metals such as e.g. Pt, Au and Ag, but can also be less noble metals such as copper or aluminum. Electrodes can be composed of multiple layers each comprising any of the aforementioned electrode materials. This can be useful to improve mechanical compliance and/or adhesion with EAPs to which the electrodes are attached. Electrodes can be applied using conventional deposition techniques such as coating techniques (e.g. spincoating, doctor blade, spray coating etc. for the organic or composite material electrodes) or evaporation techniques or sputter techniques (e.g. for metal electrodes). Electrodes can have various thicknesses including but not limited to millimeter range, micrometer range, nanometer range thicknesses.

Preferably, the electrodes used may be stretchable so that they follow the deformation of the EAP material layer. Materials suitable for the electrodes are also known, and may for example be selected from the group consisting of thin metal films, such as gold, copper, or aluminum or organic conductors such as carbon black, carbon nanotubes, graphene, poly-aniline (PANI), poly(3,4-ethylenedioxythiophene) (PEDOT), e.g. poly(3,4-ethylenedioxythiophene) poly(styrenesulfonate) (PEDOT:PSS). Metalized polyester films may also be used, such as metalized polyethylene terephthalate (PET), for example using an aluminum coating.

The materials for the different layers will be selected for example taking account of the elastic moduli (Young's moduli) of the different layers.

Additional layers to those discussed above may be used to adapt the electrical or mechanical behavior of the device, such as additional polymer layers.

The EAP devices are typically electric field driven devices, but ionic devices may also be used. Ionic devices may be based on ionic polymer - metal composites (IPMCs) or conjugated polymers. An ionic polymer - metal composite (IPMC) is a synthetic composite nanomaterial that displays artificial muscle behavior under an applied voltage or electric field.

IPMCs are composed of an ionic polymer like Nafion or Flemion whose surfaces are chemically plated or physically coated with conductors such as platinum or gold, or carbon-based electrodes. Under an applied voltage, ion migration and redistribution due to the imposed voltage across a strip of IPMCs result in a bending deformation. The polymer is a solvent swollen ion-exchange polymer membrane. The field causes cations travel to cathode side together with water. This leads to reorganization of hydrophilic clusters and to polymer expansion. Strain in the cathode area leads to stress in rest of the polymer matrix resulting in bending towards the anode. Reversing the applied voltage inverts the bending.

If the electrodes are arranged in a non-symmetric configuration, the imposed voltage can induce all kinds of deformations such as twisting, rolling, torsioning, turning, and non-symmetric bending deformation.

The device may be used as a single actuator, or else there may be a line or array of the devices, for example to provide control of a 2D or 3D contour.

The invention can be applied in many EAP applications, including examples where a passive matrix array of actuators is of interest, in particular as a result of the threshold function described above for some actuator examples.

In many applications the main function of the product relies on the (local) manipulation of human tissue, or the actuation of tissue contacting interfaces. In such applications EAP actuators provide unique benefits mainly because of the small form factor, the flexibility and the high energy density. Hence EAP's can be easily integrated in soft, 3D-shaped and / or miniature products and interfaces. Examples of such applications are:
Skin cosmetic treatments such as skin actuation devices in the form of EAP-based skin patches which apply a constant or cyclic stretch to the skin in order to tension the skin or to reduce wrinkles;
Respiratory devices with a patient interface mask which has an EAP-based active cushion or seal, to provide an alternating normal pressure to the skin which reduces or prevents facial red marks;
Electric shavers with an adaptive shaving head. The height of the skin contacting surfaces can be adjusted using EAP actuators in order to influence the balance between closeness and irritation;
Oral cleaning devices such as an air floss with a dynamic nozzle actuator to improve the reach of the spray, especially in the spaces between the teeth. Alternatively, toothbrushes may be provided with activated tufts;
Consumer electronics devices or touch panels which provide local haptic feedback via an array of EAP transducers which is integrated in or near the user interface;
Catheters with a steerable tip to enable easy navigation in tortuous blood vessels.

Another category of relevant application which benefits from EAP actuators relates to the modification of light. Optical elements such as lenses, reflective surfaces, gratings etc. can be made adaptive by shape or position adaptation using EAP actuators. Here the benefits of EAPs are for example the lower power consumption.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

## Claims

1. A method of controlling a device having an actuation member comprising an electroactive material capable of deforming upon driving with a control signal, the method comprising the steps of:
generating an actuation signal as a control signal for causing an actuation of the actuation member wherein the actuation has a maximum first actuation frequency;
generating a vibration signal as a control signal for causing a vibration of the actuation member for reducing friction, wherein the vibration has a frequency greater than the first actuation frequency; and
supplying the actuation signal to at least part of the electroactive material and supplying the vibration signal to at least part of the electroactive material.

2. A method as claimed in claim 1, wherein the device comprises a substrate against which the actuation member is positioned, wherein the vibration of the actuation member is to reduce friction between the substrate and the actuation member.

3. A method as claimed in claim 2, wherein the substrate comprises a further actuation member as claimed in claim 1 which can be driven by the control signal or another control signal.

4. A method as claimed in claim 1, wherein the device is to be moved along an external surface in use, wherein the vibration of the actuation member is to reduce friction between the actuation member and the external surface.

5. A method as claimed in any preceding claim, wherein, in a drive period, the actuation signal and the vibration signal are supplied to the electroactive material such that they overlap in the drive period.

6. A method as claimed in any preceding claim, wherein the actuation signal comprises a non-oscillating signal and the vibration signal comprises:
a pulsed signal including one or more pulses, and/or
an oscillating signal.

7. A method as claimed in any preceding claim, wherein:
the actuation signal comprises an actuation signal amplitude and the vibration signal comprises a vibration signal amplitude and the vibration signal amplitude is smaller than 20%, or smaller than 10% or smaller than 5% of the actuation signal amplitude; or
the actuation signal is an oscillating signal comprising an actuation signal frequency and the vibration signal comprises a vibration signal frequency that is higher than the actuation signal frequency.

8. A method as claimed in any preceding claim, wherein the vibration signal comprises a vibration signal frequency which is chosen to be: < 1 MHz, <100kHz, <10kHz, or <1 kHz and wherein optionally the vibration signal comprises at least one vibration signal frequency that is equal to a resonance frequency or eigenfrequency of the actuation member.

9. A method as claimed in any preceding claim, comprising the steps of:
in a first operating mode, supplying the actuation signal and the vibration signal to the electroactive material; and
in a second operating mode, supplying only an actuation signal and no vibration signal to the electroactive material.

10. A method as claimed in any preceding claim, wherein the actuation signal comprises an actuation signal amplitude, the vibration signal comprises a vibration signal amplitude and a vibration signal frequency, the method comprising the steps of:
selecting the amplitude of the actuation signal to provide a desired level of the actuation; and/or
selecting the amplitude of the vibration signal to provide a desired level of the vibration; and/or
selecting the frequency of the vibration signal to cause the vibration to be a resonant vibration.

11. Computer program product comprising computer code stored on a computer readable medium or downloadable from a communications network, the computer code, when executed on a computer, implementing a method of any one of claims 1 to 10.

12. A device comprising:
an actuation member comprising an electroactive material (30) capable of deforming upon driving with a control signal: and
a controller (34) configured to implement the steps of any one of the methods of claims 1 to 10.

13. A device as claimed in claim 12, further comprising:
a first electrode arrangement configured to receive the actuation signal and therewith to supply it to a first part of the electroactive material; and
a second electrode arrangement configured to receive the vibration signal and therewith to supply it to a further part of the electroactive material that is the same as or different from the first part of the electroactive material.

14. A device as claimed in claim 12 or 13, comprising:
a body for guided movement along an internal guide or an external conduit, the body comprising the actuation member and optionally the internal guide or external conduit comprising the substrate; or
an internal guide or external conduit for guided movement of a body, the internal guide or external conduit comprising the actuation member and optionally the body comprising the substrate.

15. A device as claimed in claim 12, 13 or 14 wherein the electroactive material comprises one or more of the following: a dielectric elastomer, a piezoelectric polymer, a ferroelectric relaxor polymer or ferroelectric polymer, or an electrostrictive polymer.

## Patentansprüche

1. Verfahren zur Steuerung einer Vorrichtung, die ein Betätigungselement umfasst, das ein elektroaktives Material umfasst, das sich beim Ansteuern mit einem Steuersignal verformen kann, wobei das Verfahren die folgenden Schritte umfasst:
Erzeugen eines Betätigungssignals als ein Steuersignal zum Bewirken einer Betätigung des Betätigungselements, wobei die Betätigung eine maximale erste Betätigungsfrequenz aufweist;
Erzeugen eines Vibrationssignals als Steuersignal zum Bewirken einer Vibration des Betätigungselements zum Reduzieren der Reibung, wobei die Vibration eine Frequenz aufweist, die größer als die erste Betätigungsfrequenz ist; und
Zuführen des Betätigungssignals zu mindestens einem Teil des elektroaktiven Materials und Zuführen des Vibrationssignals zu mindestens einem Teil des elektroaktiven Materials.

2. Verfahren nach Anspruch 1, wobei die Vorrichtung ein Substrat umfasst, gegen das das Betätigungselement positioniert ist, wobei die Vibration des Betätigungselements die Reibung zwischen dem Substrat und dem Betätigungselement reduzieren soll.

3. Verfahren nach Anspruch 2, wobei das Substrat ein weiteres Betätigungselement nach Anspruch 1 umfasst, das durch das Steuersignal oder ein anderes Steuersignal angesteuert werden kann.

4. Verfahren nach Anspruch 1, wobei die Vorrichtung im Gebrauch entlang einer Außenfläche bewegt werden soll, wobei die Vibration des Betätigungselements die Reibung zwischen dem Betätigungselement und der Außenfläche reduzieren soll.

5. Verfahren nach einem der vorstehenden Ansprüche, wobei in einer Ansteuerperiode das Betätigungssignal und das Vibrationssignal dem elektroaktiven Material so zugeführt werden, dass sie sich in der Ansteuerperiode überlappen.

6. Verfahren nach einem der vorstehenden Ansprüche, wobei das Betätigungssignal ein nicht oszillierendes Signal umfasst und das Vibrationssignal Folgendes umfasst:
ein gepulstes Signal, das einen oder mehrere Impulse beinhaltet, und/oder
ein oszillierendes Signal.

7. Verfahren nach einem der vorstehenden Ansprüche, wobei:
das Betätigungssignal eine Betätigungssignalamplitude umfasst und das Vibrationssignal eine Vibrationssignalamplitude umfasst und die Vibrationssignalamplitude kleiner als 20 % oder kleiner als 10 % oder kleiner als 5 % der Betätigungssignalamplitude ist; oder
das Betätigungssignal ein oszillierendes Signal ist, das eine Betätigungssignalfrequenz umfasst, und das Vibrationssignal eine Vibrationssignalfrequenz umfasst, die höher als die Betätigungssignalfrequenz ist.

8. Verfahren nach einem der vorstehenden Ansprüche, wobei das Vibrationssignal eine Vibrationssignalfrequenz umfasst, die gewählt wird als: <1 MHz, <100 kHz, <10 kHz oder <1 kHz und wobei das Vibrationssignal gegebenenfalls mindestens eine Vibrationssignalfrequenz umfasst, die gleich einer Resonanzfrequenz oder Eigenfrequenz des Betätigungselements ist.

9. Verfahren nach einem der vorstehenden Ansprüche, umfassend die folgenden Schritte:
Zuführen des Betätigungssignals und des Vibrationssignals an das elektroaktive Material in einem ersten Betriebsmodus; und
Zuführen nur eines Betätigungssignals und keines Vibrationssignals an das elektroaktive Material in einem zweiten Betriebsmodus.

10. Verfahren nach einem der vorstehenden Ansprüche, wobei das Betätigungssignal eine Betätigungssignalamplitude umfasst, das Vibrationssignal eine Vibrationssignalamplitude und eine Vibrationssignalfrequenz umfasst, wobei das Verfahren die folgenden Schritte umfasst:
Auswählen der Amplitude des Betätigungssignals, um ein gewünschtes Betätigungsniveau bereitzustellen; und/oder
Auswählen der Amplitude des Vibrationssignals, um ein gewünschtes Vibrationsniveau bereitzustellen; und/oder
Auswählen der Frequenz des Vibrationssignals, um zu bewirken, dass die Vibration eine Resonanzvibration ist.

11. Computerprogrammprodukt, das Computercode umfasst, der auf einem computerlesbaren Medium gespeichert ist oder von einem Kommunikationsnetz herunterladbar ist, wobei der Computercode bei Ausführung auf einem Computer ein Verfahren nach einem der Ansprüche 1 bis 10 implementiert.

12. Vorrichtung, umfassend:
ein Betätigungselement, umfassend ein elektroaktives Material (30), das sich beim Ansteuern mit einem Steuersignal verformen kann;
eine Steuerung (34), die konfiguriert ist, um die Schritte eines der Verfahren der Ansprüche 1 bis 10 zu implementieren.

13. Vorrichtung nach Anspruch 12, weiter umfassend:
eine erste Elektrodenanordnung, die konfiguriert ist, um das Betätigungssignal zu empfangen und es damit einem ersten Teil des elektroaktiven Materials zuzuführen; und
eine zweite Elektrodenanordnung, die konfiguriert ist, um das Vibrationssignal zu empfangen und es damit einem weiteren Teil des elektroaktiven Materials zuzuführen, der dem ersten Teil des elektroaktiven Materials entspricht oder davon verschieden ist.

14. Vorrichtung nach Anspruch 12 oder 13, umfassend:
einen Körper zur geführten Bewegung entlang einer Innenführung oder einer Außenleitung, wobei der Körper das Betätigungselement und gegebenenfalls die Innenführung oder die Außenleitung, die das Substrat umfasst, umfasst; oder
eine Innenführung oder Außenleitung zur geführten Bewegung eines Körpers, wobei die Innenführung oder Außenleitung das Betätigungselement umfasst und gegebenenfalls der Körper das Substrat umfasst.

15. Vorrichtung nach Anspruch 12, 13 oder 14, wobei das elektroaktive Material eines oder mehrere der folgenden Elemente umfasst: ein dielektrisches Elastomer, ein piezoelektrisches Polymer, ein ferroelektrisches Relaxorpolymer oder ein ferroelektrisches Polymer oder ein elektrostriktives Polymer.

## Revendications

1. Procédé de commander d'un dispositif ayant un élément d'actionnement comprenant une matière électro-active susceptible de déformation lors d'un pilotage avec un signal de commande, le procédé comprenant les étapes de :
production d'un signal d'actionnement en tant que signal de commande pour provoquer un actionnement de l'élément d'actionnement dans lequel l'actionnement a une première fréquence d'actionnement maximale ;
production d'un signal de vibration comme en tant que signal de commande pour provoquer une vibration de l'élément d'actionnement pour réduire la friction, dans lequel la vibration a une fréquence plus grande que la première fréquence d'actionnement ; et
fourniture du signal d'actionnement à au moins partie de la matière électro-active et fourniture du signal de vibration à au moins une partie de la matière électro-active.

2. Procédé selon la revendication 1, dans lequel le dispositif comprend un substrat contre lequel l'élément d'actionnement est positionné, dans lequel la vibration de l'élément d'actionnement doit réduire la friction entre le substrat et l'élément d'actionnement.

3. Procédé selon la revendication 2, dans lequel le substrat comprend un élément d'actionnement supplémentaire comme revendiqué dans la revendication 1 qui peut être piloté par le signal de commande ou un autre signal de commande.

4. Procédé selon la revendication 1, dans lequel le dispositif doit être déplacé le long d'une surface externe en fonctionnement, dans lequel la vibration de l'élément d'actionnement doit réduire la friction entre l'élément d'actionnement et la surface externe.

5. Procédé selon n'importe quelle revendication précédente, dans lequel, dans une période de pilotage, le signal d'actionnement et le signal de vibration sont fournis à la matière électro-active de sorte qu'ils se chevauchent dans la période de pilotage.

6. Procédé selon n'importe quelle revendication précédente, dans lequel le signal d'actionnement comprend un signal non oscillant et le signal de vibration comprend :
un signal pulsé incluant une ou plusieurs impulsions, et/ou
un signal oscillant.

7. Procédé selon n'importe quelle revendication précédente, dans lequel :
le signal d'actionnement comprend une amplitude de signal d'actionnement et le signal de vibration comprend une amplitude de signal de vibration et l'amplitude de signal de vibration est plus petite que 20 %, ou plus petite que 10 % ou plus petite que 5 % de l'amplitude de signal d'actionnement ; ou
le signal d'actionnement est un signal oscillant comprenant une fréquence de signal d'actionnement et le signal de vibration comprend une fréquence de signal de vibration qui est plus élevée que la fréquence de signal d'actionnement.

8. Procédé selon n'importe quelle revendication précédente, dans lequel le signal de vibration comprend une fréquence de signal de vibration qui est choisie pour être : < 1 MHz, < 100 kHz, < 10 kHz, ou < 1 kHz et dans lequel de manière facultative le signal de vibration comprend au moins une fréquence de signal de vibration qui est égale à une fréquence de résonance ou une fréquence propre de l'élément d'actionnement.

9. Procédé selon n'importe quelle revendication précédente, comprenant les étapes de :
dans un premier mode de fonctionnement, fourniture du signal d'actionnement et du signal de vibration à la matière électro-active ; et
dans un second mode de fonctionnement, fourniture seulement d'un signal d'actionnement et d'aucun signal de vibration à la matière électro-active.

10. Procédé selon n'importe quelle revendication précédente, dans lequel le signal d'actionnement comprend une amplitude de signal d'actionnement, le signal de vibration comprend une amplitude de signal de vibration et une fréquence de signal de vibration, le procédé comprenant les étapes de :
la sélection de l'amplitude du signal d'actionnement pour fournir un niveau souhaité de l'actionnement ; et/ou
la sélection de l'amplitude du signal de vibration pour fournir un niveau souhaité de la vibration ; et/ou
la sélection de la fréquence du signal de vibration pour amener la vibration à être une vibration résonante.

11. Produit formant programme informatique comprenant un code informatique stocké sur un support lisible par ordinateur ou téléchargeable à partir d'un réseau de communication, le code informatique, quand il est exécuté sur un ordinateur, mettant en oeuvre un procédé selon n'importe laquelle des revendications 1 à 10.

12. Dispositif comprenant:
un élément d'actionnement comprenant une matière électro-active (30) susceptible de déformation lors d'un pilotage avec un signal de commande ; et
une unité de commande (34) configurée pour mettre en oeuvre les étapes de n'importe lequel des procédés des revendications 1 à 10.

13. Dispositif selon la revendication 12, comprenant en outre :
un premier agencement d'électrode configuré pour recevoir le signal d'actionnement et avec celui-ci le fournir à une première partie de la matière électro-active ; et
un second agencement d'électrode configuré pour recevoir le signal de vibration et avec celui-ci le fournir à une partie supplémentaire de la matière électro-active qui est la même que ou différente de la première partie de la matière électro-active.

14. Dispositif selon la revendication 12 ou 13, comprenant :
un corps pour un mouvement guidé le long d'un guide interne ou d'un conduit externe, le corps comprenant l'élément d'actionnement et de manière facultative le guide interne ou le conduit externe comprenant le substrat ; ou
un guide interne ou un conduit externe pour un mouvement guidé d'un corps, le guide interne ou le conduit externe comprenant l'élément d'actionnement et de manière facultative le corps comprenant le substrat.

15. Dispositif selon la revendication 12, 13 ou 14, dans lequel la matière électro-active comprend un ou plusieurs de ce qui suit : un élastomère diélectrique, un polymère piézoélectrique, un polymère relaxor ferroélectrique ou un polymère ferroélectrique, ou un polymère électrostrictif.
